(19) Europäisches Patentamt
European Patent Office
Office européen des brevets

(11) Veröffentlichungsnummer: **0 026 437**
**B1**

(12) # EUROPÄISCHE PATENTSCHRIFT

(45) Veröffentlichungstag der Patentschrift: **08.06.83**

(51) Int. Cl.³: **C 07 C 69/743,** A 01 N 53/00

(21) Anmeldenummer: **80105719.1**

(22) Anmeldetag: **24.09.80**

(54) **3-(2,3-Dichlor-3,3-difluor-prop-1-en-1-yl)2,2-dimethyl-cyclopropancarbonsäure-fluor-benzylester, Verfahren zu ihrer Herstellung sowie ihre Verwendung in Schädlingsbekämpfungsmitteln.**

(30) Priorität: **02.10.79 DE 2939913**

(43) Veröffentlichungstag der Anmeldung: **08.04.81 Patentblatt 81/14**

(45) Bekanntmachung des Hinweises auf die Patenterteilung: **08.06.83 Patentblatt 83/23**

(84) Benannte Vertragsstaaten: **AT BE CH DE FR GB IT LI NL SE**

(56) Entgegenhaltungen:
**EP-A-0 001 064**
**EP-A-0 010 879**
**DE-A-2 650 534**
**DE-A-2 721 185**
**GB-A-2 000 764**

**Die Akte enthält technische Angaben, die nach dem Eingang der Anmeldung eingereicht wurden und die nicht in dieser Patentschrift enthalten sind.**

(73) Patentinhaber: **BAYER AG, Zentralbereich Patente, Marken und Lizenzen, D-5090 Leverkusen 1, Bayerwerk (DE)**

(72) Erfinder: **Fuchs, Rainer, Dr., Roeberstrasse 8, D-5600 Wuppertal 1 (DE)**
Erfinder: **Naumann, Klaus, Dr., Richard-Wagner-Strasse 9, D-5090 Leverkusen 1 (DE)**
Erfinder: **Lantzsch, Reinhard, Dr., Heymannstrasse 32, D-5090 Leverkusen 1 (DE)**
Erfinder: **Hagemann, Hermann, Dr., Roggendorfstrasse 55, D-5000 Köln 80 (DE)**
Erfinder: **Hammann, Ingeborg, Dr., Belfortstrasse 9, D-5000 Köln 80 (DE)**
Erfinder: **Homeyer, Bernhard, Dr., Obere Strasse 28, D-5090 Leverkusen 3 (DE)**
Erfinder: **Behrenz, Wolfgang, Dr., Untergründemich 14, D-5063 Overath (DE)**
Erfinder: **Stendel, Wilhelm, Dr., In den Birken 55, D-5600 Wuppertal 1 (DE)**

**EP 0 026 437 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# 3-(2,3-Dichlor-3,3-difluorprop-1-en-1-yl)-2,2-dimethylcyclopropancarbonsäurefluorbenzylester, Verfahren zu ihrer Herstellung sowie ihre Verwendung in Schädlingsbekämpfungsmitteln

Die Erfindung betrifft neue 3-(2,3-Dichlor-3,3-difluorprop-1-en-1-yl)-2,2-dimethylcyclopropancarbonsäurefluorbenzylester, ein Verfahren zu ihrer Herstellung sowie ihre Verwendung in Schädlingsbekämpfungsmitteln, insbesondere in Insektiziden und Akariziden.

Es ist bekannt, dass bestimmte substituierte Cyclopropancarbonsäureester, wie z.B. 3-(2-Methylprop-1-en-1-yl)- und 3-(2,2-Dichlorvinyl)-2,2-dimethylcyclopropancarbonsäure-(3-phenoxybenzyl)ester (Phenothrin bzw. Permethrin) insektizide und akarizide Wirkung besitzen (vgl. GB-PS Nrn. 1243858 und 1413491).

Die Wirkung dieser Verbindungen ist jedoch, insbesondere bei niedrigen Wirkstoffkonzentrationen und Aufwandmengen, nicht immer zufriedenstellend.

Es wurden nun neue 3-(2,3-Dichlor-3,3-difluorprop-1-en-1-yl)-2,2-dimethylcyclopropancarbonsäurefluorbenzylester der Formel I

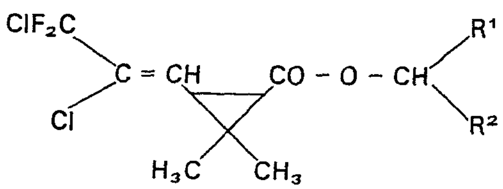

(I)

gefunden, in welcher

$R^1$ für Wasserstoff, Cyano, Alkyl, Alkenyl oder Alkinyl, letztere jeweils mit bis zu 4 Kohlenstoffatomen, steht, und

$R^2$ für einen durch Fluor und/oder gegebenenfalls fluorsubstituiertes Phenoxy substituierten Phenylrest steht, mit der Massgabe, dass der Rest $R^2$ insgesamt wenigstens einen Fluorsubstituenten enthält.

Man erhält die neuen Verbindungen der Formel I, wenn man 3-(2,3-Dichlor-3,3-difluorprop-1-en-1-yl)-2,2-dimethylcyclopropancarbonsäure der Formel II

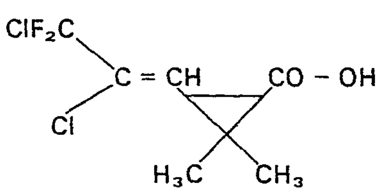

(II)

oder reaktionsfähige Derivate derselben, mit Benzylalkoholen der Formel III

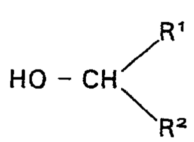

(III)

in welcher

$R^1$ und $R^2$ die oben angegebenen Bedeutungen haben, oder mit reaktionsfähigen Derivaten derselben, gegebenenfalls in Gegenwart von Säureakzeptoren und/oder Katalysatoren und gegebenenfalls unter Verwendung von Verdünnungsmitteln umsetzt.

Die neuen 3-(2,3-Dichlor-3,3-difluorprop-1-en-1-yl)-2,2-dimethylcyclopropancarbonsäurefluorbenzylester der Formel I zeichnen sich durch hohe insektizide und akarizide Wirksamkeit aus.

Überraschenderweise zeigen die erfindungsgemässen Verbindungen der Formel I eine erheblich höhere insektizide und akarizide Wirkung als aus dem Stand der Technik bekannte Verbindungen analoger Konstitution und gleicher Wirkungsrichtung.

Die allgemeine Formel I schliesst die verschiedenen möglichen Stereoisomeren und optisch aktiven Isomeren sowie deren Mischungen mit ein.

Gegenstand der Erfindung sind vorzugsweise Verbindungen der Formel I, in welcher

$R^1$ für Wasserstoff oder Cyano steht, und

$R^2$ für Pentafluorphenyl, 4-Fluor-3-phenoxyphenyl, 3-(4-Fluorphenoxy)phenyl oder 4-Fluor-3-(4-fluorphenoxy)phenyl steht.

In einer bevorzugten Variante a des Herstellungsverfahrens für die Verbindungen der Formel I wird 3-(2,3-Dichlor-3,3-difluorprop-1-en-1-yl)-2,2-dimethylcyclopropancarbonsäurechlorid der Formel IIa

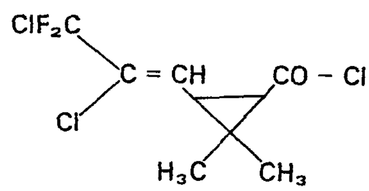

(IIa)

mit Benzylalkoholen der Formel III (oben) in Gegenwart von Säureakzeptoren und unter Verwendung von Verdünnungsmitteln umgesetzt.

In einer weiteren bevorzugten Verfahrensvariante b, insbesondere zur Herstellung von Verbindungen der Formel I, in welcher $R^1$ für Cyano und $R^2$ für fluorsubstituiertes Phenoxyphenyl steht, wird das Säurechlorid der Formel IIa (oben) mit entsprechenden Phenoxybenzaldehyden der Formel IV

$$OHC{-}R^2 \qquad (IV)$$

in welcher

$R^2$ für fluorsubstituiertes Phenoxyphenyl steht, und wenigstens der äquimolaren Menge eines Alkalicyanids (Natrium- oder Kaliumcyanid) in Gegenwart von Wasser und eines mit Wasser nicht mischbaren organischen Lösungsmittels und gegebenenfalls in Gegenwart eines Katalysators umgesetzt.

Als weitere reaktionsfähige Derivate der Carbonsäure der Formel II sind deren Niederalkylester zu nennen, welche mit Alkoholen der Formel III nach üblichen Methoden umgesetzt werden können.

Alkali-, Erdalkali- oder Ammoniumsalze der Carbonsäure der Formel II können mit Benzylhalogeniden, welche sich von den Benzylalkoholen der

Formel III ableiten, ebenfalls zu Verbindungen der Formel I umgesetzt werden.

Verwendet man als Ausgangsstoffe bei der Verfahrensvariante a beispielsweise Pentafluorbenzylalkohol und bei Varainte b 4-Fluor-3-(4-fluorphenoxy)benzaldehyd, so können die Reaktionen bei den beiden Verfahrensvarianten durch folgende Formelschemata skizziert werden:

(a)

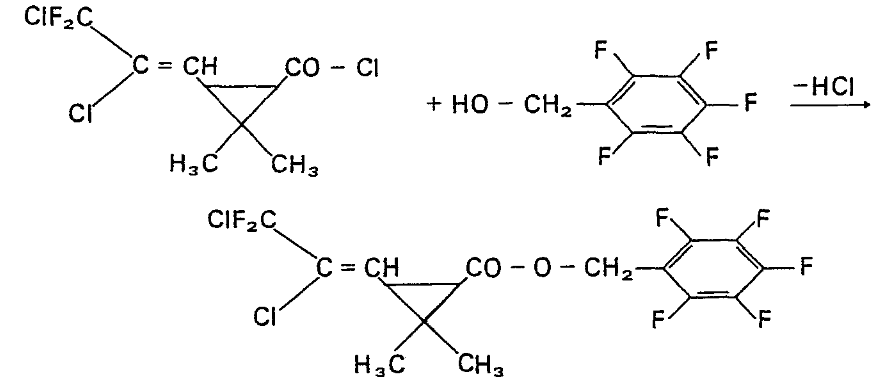


(b)

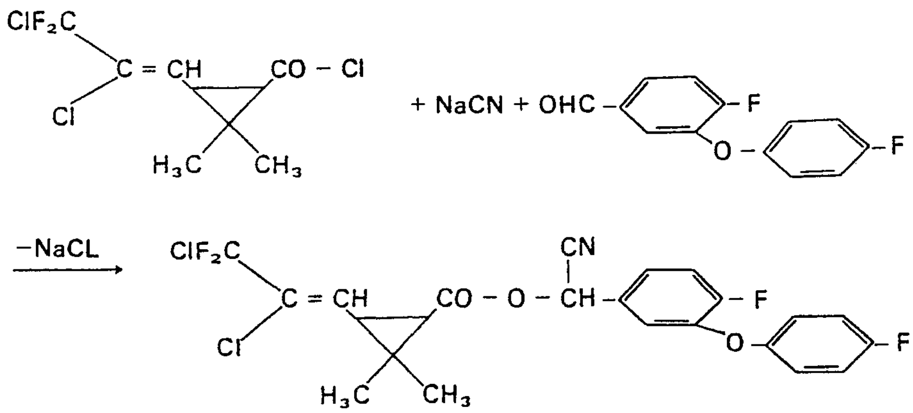


Die als Ausgangsverbindung zu verwendende 3-(2,3-Dichlor-3,3-difluorprop-1-en-1-yl)-2,2-dimethylcyclopropancarbonsäure ist bereits bekannt (vgl. DE-OS Nr. 2802962 und GB-PS Nr. 2000764).

Das Säurechlorid der Formel IIa erhält man daraus auf übliche Weise, beispielsweise durch Umsetzung mit Thionylchlorid, gegebenenfalls in Gegenwart eines Verdünnungsmittels, wie z.B. Tetrachlorkohlenstoff, bei Temperaturen zwischen 10 und 100° C.

Die weiter als Ausgangsstoffe zu verwendenden Benzylalkohole sind durch Formel III definiert. Vorzugsweise stehen darin $R^1$ und $R^2$ für diejenigen Reste, welche bereits bei der Definition der Reste $R^1$ und $R^2$ in Formel I als bevorzugt genannt wurden.

Als Beispiele für die Ausgangsverbindungen der Formel III seien genannt:

Pentafluorbenzylalkohol, 4-Fluor-3-phenoxybenzylalkohol, 3-(4-Fluorphenoxy)benzylalkohol, 4-Fluor-3-(4-fluorphenoxy)benzylalkohol und 3-(4-Fluorphenoxy)-α-cyanobenzylalkohol.

Die Ausgangsverbindungen der Formel III sind bereits bekannt (vgl. GB-PS Nr. 1078511, DE-OS Nrn. 2621433, 2709264 und 2739854).

Die als Ausgangsstoffe verwendbaren Phenoxybenzaldehyde sind durch Formel IV definiert. Vorzugsweise steht darin $R^2$ für diejenigen Reste, welche bereits bei der Definition von $R^2$ in Formel I als bevorzugt genannt wurden.

Als Beispiele seien genannt:

4-Fluor-3-phenoxybenzaldehyd, 3-(4-Fluorphenoxy)benzaldehyd und 4-Fluor-3-(4-fluorphenoxy)benzaldehyd.

Die Phenoxybenzaldehyde der Formel IV sind bereits bekannt (vgl. DE-OS Nrn. 2621433, 2709264 und 2739854).

Das Verfahren zur Herstellung der neuen Verbindungen der Formel I wird in allen Varianten, vorzugsweise unter Verwendung von Verdünnungsmitteln, durchgeführt. Als Verdünnungsmittel kommen praktisch alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petroläther, Benzin, Ligroin, Benzol, Toluol, Xylol, Methylenchlorid, Äthylenchlorid, Chloroform, Tetrachlorkohlenstoff, Chlorbenzol und o-Dichlorbenzol, Äther, wie Diäthyl- und Dibutyläther, Glycoldimethyläther und Diglycoldimethyläther, Tetrahydrofuran, und Dioxan, Ketone, wie Aceton, Methyläthyl-, Methylisopropyl- und Methylisobutylketon, Ester, wie Essigsäuremethylester und -äthylester, Nitrile, wie z.B. Acetonitril und Propionitril, Amide, wie z.B. Dimethylformamid, Dimethylacetamid und N-Methylpyrrolidon, sowie Dimethylsulfoxid, Tetramethylensulfon und Hexamethylphosphorsäuretriamid.

Die Variante a des erfindungsgemässen Verfahrens wird vorzugsweise in Gegenwart von Säureakzeptoren durchgeführt. Als Säureakzeptoren können die üblichen Säurebindemittel Verwendung finden. Besonders bewährt haben sich Alkalicarbonate und -alkoholate, wie Natrium- und Kaliumcarbonat, Natrium- und Kaliummethylat bzw. -äthylat, ferner aliphatische, aromatische oder heterocyclische Amine, beispielsweise Triäthylamin, Trimethylamin, Dimethylanilin, Dimethylbenzylamin, Pyridin, Diazabicyclooctan, Diazabicyclononan und Diazabicycloundecen.

Die Variante b des erfindungsgemässen Verfahrens wird in Gegenwart von Wasser und eines der obengenannten organischen Lösungsmittel, soweit mit Wasser nicht mischbar, durchgeführt. Hierfür sind insbesondere die obengenannten Kohlenwasserstoffe geeignet.

Als Katalysatoren werden bei der Verfahrensvariante b vorzugsweise Verbindungen verwendet, welche zum Transfer von Anionen aus Wasser in organische Lösungsmittel geeignet sind. Beispiele hierfür sind Benzyltriäthylammoniumhydrogensulfat, Tetrabutylammoniumbromid und Methyltrioctylammoniumchlorid (Aliquat 336).

In allen Verfahrensvarianten kann die Reaktionstemperatur innerhalb eines grösseren Bereichs variiert werden. Im allgemeinen arbeitet man zwischen 0 und 100° C, vorzugsweise bei 10 bis 50° C.

Das erfindungsgemässe Verfahren wird im allgemeinen bei Normaldruck durchgeführt.

Zur Durchführung des erfindungsgemässen Verfahrens werden die Ausgangsstoffe gewöhnlich in äquimolaren Mengen eingesetzt. Ein Überschuss der einen oder anderen Reaktionskomponente bringt keine wesentlichen Vorteile. Die Ausgangsstoffe werden in geeigneten Verdünnungsmitteln zusammen gegeben und, gegebenenfalls nach Zugabe eines Säureakzeptors und/oder eines Katalysators, bis zum Reaktionsende gerührt.

Die Aufarbeitung kann nach üblichen Methoden durchgeführt werden, beispielsweise indem man das Reaktionsgemisch gegebenenfalls mit Wasser und/oder einem mit Wasser nicht mischbaren organischen Lösungsmittel, wie z.B. Toluol, verdünnt, die organische Phase abtrennt, mit Wasser wäscht, trocknet, filtriert und vom Filtrat das Lösungsmittel unter vermindertem Druck und bei mässig erhöhter Temperatur sorgfältig abdestilliert (andestilliert).

Die Wirkstoffe eignen sich bei guter Pflanzenverträglichkeit und günstiger Warmblütertoxizität zur Bekämpfung von tierischen Schädlingen, insbesondere Insekten, Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den obenerwähnten Schädlingen gehören:

Aus der Ordnung der *Isopoda*, z.B. *Oniscus asellus, Armadillidium vulgare, Porcellio scaber.*

Aus der Ordnung der *Diplopoda*, z.B. *Blaniulus guttulatus.*

Aus der Ordnung der *Chilopoda*, z.B. *Geophilus carpophagus, Scutigera spec.*

Aus der Ordnung der *Symphyla*, z.B. *Scutigerella immaculata.*

Aus der Ordnung der *Tysanura*, z.B. *Lepisma saccharina.*

Aus der Ordnung der *Collembola*, z.B. *Onychiurus armatus.*

Aus der Ordnung der *Orthoptera*, z.B. *Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.*

Aus der Ordnung der *Dermaptera*, z.B. *Forficula auricularia.*

Aus der Ordnung der *Isoptera*, z.B. *Reticulitermes spp.*

Aus der Ordnung der *Anoplura*, z.B. *Phylloxera vastatrix, Pemphigus spp., Pediculus humanus corporis, Haematopinus spp., Linognathus spp.*

Aus der Ordnung der *Mallophaga*, z.B. *Trichodectes spp., Damalinea spp.*

Aus der Ordnung der *Thysanoptera*, z.B. *Hercinothrips femoralis, Thrips tabaci.*

Aus der Ordnung der *Heteroptera*, z.B. *Eurygaster spp., Dysdercus intermedius, Piesma quadrata, Cimex lectularius, Rhodnius prolixus, Triatoma spp.*

Aus der Ordnung der *Homoptera*, z.B. *Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.*

Aus der Ordnung der *Lepidoptera*, z.B. *Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix vididana.*

Aus der Ordnung der *Coleoptera*, z.B. *Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes crysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera*

*postica, Dermestes spp., Trodogerma spp., Anthrenus spp., Attagenus spp., Lyctus spp., Meligethes aeneus, Ptinus spp., Niptus hololeucus, Gibbium psylloides, Tribolium spp., Tnebrio molitor, Agriotes spp., Conoderus spp., Melolontha melolontha, Amphimallon solstitialis, Costelytra zealandica.*

Aus der Ordnung der *Hymenoptera*, z.B. *Diprion spp., Hoplocampa spp., Lasius spp., Monomorium pharaonis, Vespa spp.*

Aus der Ordnung der *Diptera*, z.B. *Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.*

Aus der Ordnung der *Siphonaptera*, z.B. *Xenopsylla cheopis, Ceratophyllus spp.*

Aus der Ordnung der *Arachnida*, z.B. *Scorpio maurus, Latrodectus mactans.*

Aus der Ordnung der *Acarina*, z.B. *Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.*

Die Wirkstoffe können in die üblichen Formulierungen übergeführt werden, wie Lösungen, Emulsionen, Suspensionen, Pulver, Schäume, Pasten, Granulate, Aerosole, wirkstoffimprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebelformulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosoltreibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier- und/oder schaumerzeugende Mittel kommen in Frage: z.B. nichtionogene und anionische Emulgatoren, wie Polyoxyäthylenfettsäureester, Polyoxyäthylenfettalkoholäther, z.B. Alkylarylpolyglykoläther, Alkylsulfonate, Alkylsulfate, Arylsulfonate sowie Eiweisshydrolysate; als Dispergiermittel kommen in Frage: z.B. Ligninsulfitablaugen und Methylcellulose.

Es können in den Formulierungen Haftmittel wie Carboxymethylcellulose, natürliche und synthetische pulverige, körnige und latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azolmetallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.% Wirkstoff, vorzugsweise zwischen 0,5 und 90 Gew.%.

Die Anwendung der erfindungsgemässen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.% Wirkstoff, vorzugsweise zwischen 0,000001 und 1 Gew.%, liegen.

Die Anwendung geschieht in einer den Anwendungsformen angepassten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

*Beispiel A:*

*Drosophila*-Test

Lösungsmittel: 3 Gewichtsteile Aceton

Emulgator: 1 Gewichtsteil Alkylarylpolyglykoläther

Zur Herstellung einer zweckmässigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

1 $cm^3$ der Wirkstoffzubereitung wird auf eine Filterpapierschreibe (7 cm Durchmesser) aufpipettiert. Man legt diese nass auf die Öffnung eines Glasgefässes, in dem sich 50 Taufliegen *(Drosophilia melanogaster)* befinden, und bedeckt es mit einer Glasplatte.

Nach der gewünschten Zeit bestimmt man die Abtötung in Prozent. Dabei bedeutet 100%, dass alle Fliegen abgetötet wurden; 0% bedeutet, dass keine Fliegen abgetötet wurden.

Bei diesem Test zeigen die folgenden Verbindungen der Herstellungsbeispiele überlegene Wirksamkeit gegenüber dem Stand der Technik: 4.

*Beispiel B:*

*Myzus*-Test

Lösungsmittel: 3 Gewichtsteile Aceton
Emulgator: 1 Gewichtsteil Alkylarylpolyglykoläther

Zur Herstellung einer zweckmässigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter *(Brassica oleracea)*, die stark von der Pfirsichblattlaus *(Myzus persicae)* befallen sind, werden durch Tauchen in die Wirkstoffzubereitung der gewünschten Konzentration behandelt.

Nach der gewünschten Zeit wird die Abtötung in Prozent bestimmt. Dabei bedeutet 100%, dass alle Blattläuse abgetötet wurden; 0% bedeutet, dass keine Blattläuse abgetötet wurden.

Bei diesem Test zeigen z.B. die folgenden Verbindungen der Herstellungsbeispiele überlegene Wirksamkeit gegenüber dem Stand der Technik: 1, 2.

*Beispiel C:*

*Tetranychus*-Test (resistent)

Lösungsmittel: 3 Gewichtsteile Aceton
Emulgator: 1 Gewichtsteil Alkylarylpolyglykoläther

Zur Herstellung einer zweckmässigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen *(Phaseolus vulgaris)*, die stark von allen Entwicklungsstadien der gemeinen Spinnmilbe oder Bohnenspinnmilbe *(Tetranychus urticae)* befallen sind, werden durch Tauchen in die Wirkstoffzubereitung der gewünschten Konzentration behandelt.

Nach der gewünschten Zeit wird die Abtötung in Prozent bestimmt. Dabei bedeutet 100%, dass alle Spinnmilben abgetötet wurden; 0% bedeutet, dass keine Spinnmilben abgetötet wurden.

Bei diesem Test zeigen z.B. die folgenden Verbindungen der Herstellungsbeispiele überlegene Wirksamkeit gegenüber dem Stand der Technik: 1, 2, 3.

*Beispiel D:*

Grenzkonzentrations-Test/Bodeninsekten

Testinsekt: *Agrotis segetum* (im Boden)
Lösungsmittel: 3 Gewichtsteile Aceton
Emulgator: 1 Gewichtsteil Alkylarylpolyglykoläther

Zur Herstellung einer zweckmässigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in Teile auf eine Million (=mg/l) angegeben wird. Man füllt den Boden in Töpfe und lässt diese bei Raumtemperatur stehen.

Nach 24 h werden die Testtiere in den behandelten Boden gegeben und nach weiteren 2 bis 7 d wird der Wirkungsgrad des Wirkstoffs durch Auszählen der toten und lebenden Testinsekten in Prozent bestimmt. Der Wirkungsgrad ist 100%, wenn alle Testinsekten abgetötet worden sind; er ist 0%, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 1, 2, 3, 4.

*Beispiel E:*

$LT_{100}$-Test für Dipteren

Testtiere: *Aëdes aegypti*
Zahl der Testtiere: 25
Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten geringeren Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro Quadratmeter Filterpapier verschieden hoch. Anschliessend gibt man die angegebene Anzahl der Testtiere in die Petrischale und bedeckt sie mit einem Glasdeckel.

Der Zustand der Testtiere wird laufend kontrolliert. Es wird diejenige Zeit ermittelt, welche für einen 100%igen Knockdown-Effekt notwendig ist.

Bei diesem Test zeigen z.B. die folgenden Verbindungen der Herstellungsbeispiele überlegene Wirkung gegenüber dem Stand der Technik: 3, 2, 1, 4.

*Beispiel F:*

*Boophilus microplus*-Test (resistent)

Lösungsmittel: 35 Gewichtsteile Äthylenglykolmonomethyläther

35 Gewichtsteile Nonylphenolpolyglykoläther

Zur Herstellung einer zweckmässigen Wirkstoffzubereitung vermischt man drei Gewichtsteile Wirkstoff mit sieben Gewichtsteilen des oben angegebenen Lösungsmittelgemisches und verdünnt das so erhaltene Konzentrat mit Wasser auf die gewünschte Konzentration.

10 adulte *Boophilus microplus* res. werden in die zu testende Wirkstoffzubereitung 1 min getaucht. Nach Überführung in Plastikbecher und Aufbewahrung in einem klimatisierten Raum wird der Abtötungsgrad bestimmt.

Bei diesem Test zeigen z.B. die folgenden Verbindungen der Herstellungsbeispiele überlegene Wirkung gegenüber dem Stand der Technik: 2, 3.

*Herstellungsbeispiele*

*Beispiel 1*

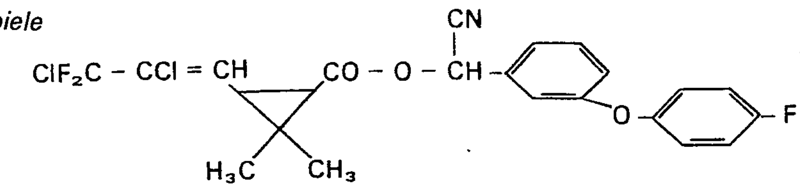

3,1 g 2,2-Dimethyl-3-(2',3'-dichlor-3',3'-difluor-1'-propenyl)cyclopropan-1-carbonsäurechlorid werden mit 100 ml Toluol und 2,67 g Cyanhydrin des 3-(4'-Fluorphenoxy)benzaldehyds vermischt. Dann tropft man bei Raumtemperatur unter Rühren 0,9 g Pyridin in 25 ml Toluol zu und rührt 4 h bei Raumtemperatur nach. Dann wird das Reaktionsgemisch mit 150 ml Wasser versetzt, die organische Phase abgetrennt und mit 100 ml Wasser gewaschen. Die organische Phase wird mit Natriumsulfat getrocknet, abfiltriert, einrotiert und bei 60° C im Hochvakuum andestilliert (ca. 1 h). Der Rückstand wiegt 4,3 g und besteht aus 2,2-Dimethyl-3-(2',3'-dichlor-3',3'-difluor-1'-propenyl)-cyclopropan-1-carbonsäure-3'-(4'-fluorphenoxy)-α-cyanobenzylester mit dem Brechungsindex $n_D^{20}$: 1,5340.

Analog Beispiel 1 erhält man:

*Beispiel 2*

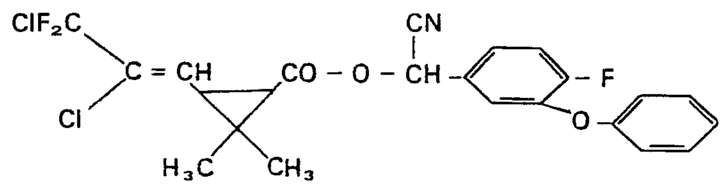

*Beispiel 3*

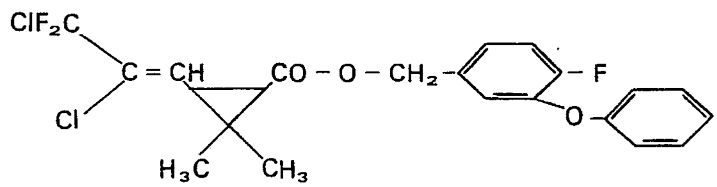

*Beispiel 4*

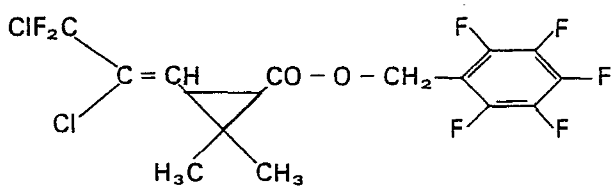

Die Ausgangsverbindungen können wie folgt hergestellt werden:

(a)

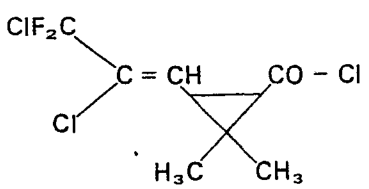

27,3 g (0,1 mol) 2,2-Dimethyl-3-(2',3'-dichlor-3',3'-difluor-1'-propenyl)cyclopropan-1-carbonsäuremethylester werden mit 135 ml 3N methanolischer Kaliumhydroxidlösung versetzt und 8 h bei Raumtemperatur gerührt. Anschliessend versetzt man mit Eiswasser und stellt mit 130 ml 10%iger Salzsäure sauer. Durch dreimaliges Extrahieren mit Methylenchlorid erhält man die 2,2-Dimethyl-3-(2',3'-dichlor-3',3'-difluor-1'-propenyl)cyclopropan-1-carbonsäure (Isomerengemisch), die mit 30 ml Thionylchlorid versetzt wird und unter Rühren 1 h auf 80° C erhitzt wird. Nach Abdestillieren des überschüssigen Thionylchlorids bei Normaldruck wird im Hochvakuum destilliert. Man erhält 21 g 2,2-Dimethyl-3-(2',3'-dichlor-3',3'-difluor-1'-propenly)cyclopropan-1-carbonsäurechlorid (Isomerengemisch) vom Siedepunkt 64° C/0,08 mbar.

(b)

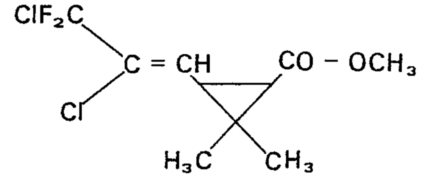

Zu einer Lösung von 183 g 30%iger Natriummethylatlösung in 500 ml Methanol wird bei Raumtemperatur innerhalb von 2 h unter Rühren die Lösung von 160 g (0,462 mol) 3,3-Dimethyl-4,6,6,7-tetrachlor-7,7-difluorönanthsäuremethylester getropft. Man rührt 2 h bei Raumtemperatur nach und erhitzt dann 8 h auf 45-50° C. Nach dem Abkühlen destilliert man einen Teil des Methanols unter vermindertem Druck ab, versetzt mit 500 ml Eiswasser und stellt neutral. Nach Extrahieren mit Methylenchlorid wird mit Natriumsulfat getrocknet und nach Abdestillieren des Lösungsmittels im Hochvakuum destilliert. Man erhält 112 g Rohprodukt vom Siedepunkt 70-95° C/0,3 mbar, das über eine Kolonne fraktioniert wird. Der reine 2,2-Dimethyl-3-(2',3'-dichlor-3',3'-difluor-1'-propenyl)cyclopropan-1-carbonsäuremethylester (Isomerengemisch von 3 Stereoisomeren) siedet bei 67-73° C/0,3 mbar.

(c)

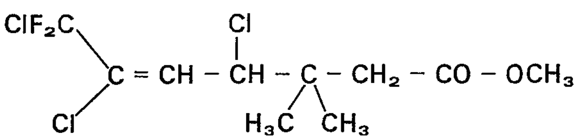

37,5 g 3,3-Dimethyl-4-pentensäuremethylester, 102 g 1,1,1,2-Tetrachlor-2,2-difluorethan, 22 g Acetonitril, 325 mg $FeCl_3 \cdot 6\ H_2O$, 255 mg Benzoin und 200 mg Dimethylaminhydrochlorid werden in einem 250-ml-VA-Autoklaven unter 6 bar Stickstoff 7 h auf 100-120° C erhitzt. Durch Destillation erhält man 3,3-Dimethyl-4,6,6,7-tetrachlor-7,7-difluorönanthsäuremethylester vom Siedepunkt 129-134° C/0,7-0,9 mbar. Brechungsindex: $n_D^{20}$: 1,4680.

## Patentansprüche

1. 3-(2,3-Dichlor-3,3-difluorprop-1-en-1-yl)-2,2-dimethylcyclopropancarbonsäurefluorbenzylester der Formel (I)

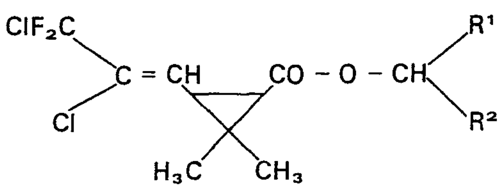

(I)

in welcher

$R^1$ für Wasserstoff, Cyano, Alkyl, Alkenyl oder Alkinyl, letztere jeweils mit bis zu 4 Kohlenstoffatomen, steht, und

$R^2$ für einen durch Fluor und/oder gegebenenfalls fluorsubstituiertes Phenoxy substituierten Phenylrest steht, mit der Massgabe, dass der Rest $R^2$ insgesamt wenigstens einen Fluorsubstituenten enthält.

2. Verfahren zur Herstellung der Verbindungen der Formel (I), dadurch gekennzeichnet, dass man 3-(2,3-Dichlor-3,3-difluorprop-1-en-1-yl)-2,2-dimethylcyclopropancarbonsäure der Formel (II)

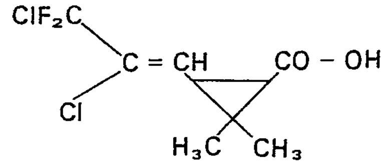

(II)

oder reaktionsfähige Derivate derselben, mit Benzylalkoholen der Formel (III)

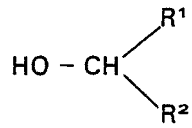

(III)

in welcher

$R^1$ und $R^2$ die oben angegebenen Bedeutungen haben, oder mit reaktionsfähigen Derivaten derselben, gegebenenfalls in Gegenwart von Säureakzeptoren und/oder Katalysatoren und gegebenenfalls unter Verwendung von Verdünnungsmitteln umsetzt.

3. Schädlingsbekämpfungsmittel, gekennzeichnet durch einen Gehalt an mindestens einer Verbindung 3-(2,3-Dichlor-3,3-difluorprop-1-en-1-yl)-2,2-dimethylcyclopropancarbonsäurefluorbenzylester der Formel (I).

4. Verwendung von Verbindungen 3-(2,3-Dichlor-3,3-difluorprop-1-en-1-yl)-2,2-dimethylcyclopropancarbonsäurefluorbenzylester der Formel (I) zur Bekämpfung von Schädlingen.

5. Verfahren zur Bekämpfung von Schädlingen, dadurch gekennzeichnet, dass man Verbindungen 3-(2,3-Dichlor-3,3-difluorprop-1-en-1-yl)-2,2-dimethylcyclopropancarbonsäurefluorbenzylester der Formel (I) auf Schädlinge und/oder ihren Lebensraum einwirken lässt.

6. Verfahren zur Herstellung von Schädlingsbekämpfungsmittel, dadurch gekennzeichnet, dass man Verbindungen 3-(2,3-Dichlor-3,3-difluorprop-1-en-1-yl)-2,2-dimethylcyclopropancarbonsäurefluorbenzylester der Formel (I) mit Streckmitteln und/oder oberflächenaktiven Mitteln vermischt.

## Revendications

1. Esters fluorobenzyliques de l'acide 3-(2,3-dichloro-3,3-difluoropropa-1-ène-1-yl)-2,2-diméthylcyclopropanecarboxylique de formule (I)

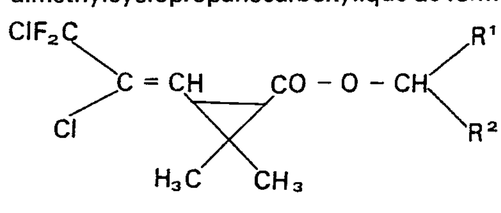

(I)

dans laquelle

$R^1$ représente l'hydrogène, un groupe cyano, alkyle, alcényle ou alcynyle, ces derniers contenant chacun jusqu'à 4 atomes de carbone, et

$R^2$ représente un groupe phényle substitué par le fluor et/ou par un groupe phénoxy lui-même éventuellement substitué par le fluor, étant spécifié que le reste $R^2$ contient au total au moins un substituant fluor.

2. Procédé de préparation des composés de formule (I), caractérisé en ce que l'on fait réagir l'acide 3-(2,3-dichloro-3,3-difluoropropa-1-ène-1-yl)-2,2-diméthylcyclopropanecarboxylique de formule (II)

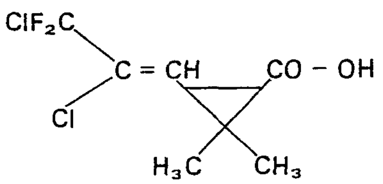
(II)

ou des dérivés réactifs de cet acide, avec des alcools benzyliques de formule (III)

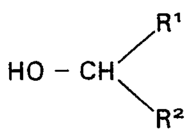
(III)

dans laquelle $R^1$ et $R^2$ ont les significations indiquées ci-dessus, ou avec des dérivés réactifs de ces alcools, éventuellement en présence d'accepteurs d'acides et/ou de catalyseurs et, le cas échéant, avec utilisation de diluants.

3. Pesticides, caractérisés en ce qu'ils contiennent au moins un ester fluorobenzylique de l'acide 3-(2,3-dichloro-3,3-difluoropropa-1-ène-1-yl)-2,2-diméthylcyclopropanecarboxylique de formule (I).

4. Utilisation des esters fluorobenzyliques de l'acide 3-(2,3-dichloro-3,3-difluoropropa-1-ène-1-yl)-2,2-diméthylcyclopropanecarboxylique de formule (I) pour combattre les parasites.

5. Procédé pour combattre les parasites, caractérisé en ce que l'on fait agir des esters fluorobenzyliques de l'acide 3-(2,3-dichloro-3,3-difluoropropa-1-ène-1-yl)-2,2-diméthylcyclopropanecarboxylique de formule (I) sur les parasites et/ou leur espace vital.

6. Procédé de préparation de pesticides, caractérisé en ce que l'on mélange des esters fluorobenzyliques de l'acide 3-(2,3-dichloro-3,3-difluoropropa-1-ène-1-yl)-2,2-diméthylcyclopropanecarboxylique de formule (I) avec des diluants et/ou des agents tensio-actifs.

## Claims

1. 3-(2,3-Dichloro-3,3-difluoroprop-1-en-1-yl)-2,2-dimethylcyclopropanecarboxylic acid fluorobenzyl esters of the formula (I)

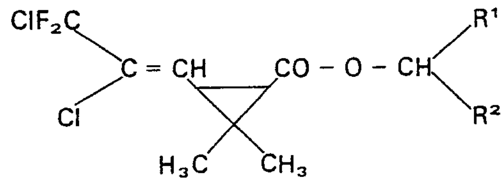
(I)

in which

$R^1$ represents hydrogen, cyano, alkyl, alkenyl or alkinyl, the latter in each case with up to 4 carbon atoms, and

$R^2$ represents a phenyl radical which is substituted by fluorine and/or optionally fluorine-substituted phenoxy, with the proviso that the radical $R^2$ in total contains at least one fluorine substituent.

2. Process for the preparation of the compounds of the formula (I), characterised in that 3-(2,3-dichloro-3,3-difluoroprop-1-en-1-yl)-2,2-dimethylcyclopropanecarboxylic acid of the formula (II)

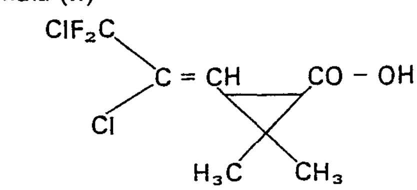
(II)

or reactive derivatives thereof, are reacted with benzyl alcohols of the formula (III)

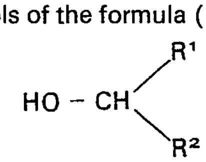
(III)

in which $R^1$ and $R^2$ have the meanings indicated above, or with reactive derivatives thereof, if appropriate in the presence of acid acceptors and/or catalysts and if appropriate using diluents.

3. Agents for combating pests, characterised in that they contain at least one compound of the formula (I), that is to say a 3-(2,3-dichloro-3,3-difluoroprop-1-en-1-yl)-2,2-dimethylcyclopropanecarboxylic acid fluorobenzyl ester.

4. Use of compounds of the formula (I), that is to say of 3-(2,3-dichloro-3,3-difluoroprop-1-en-1-yl)-2,2-dimethylcyclopropanecarboxylic acid fluorobenzyl esters, for combating pests.

5. Process for combating pests, characterised in that compounds of the formula (I), that is to say 3-(2,3-dichloro-3,3-difluoroprop-1-en-1-yl)-2,2-dimethylcyclopropanecarboxylic acid fluorobenzyl esters, are allowed to act on pests and/or their environment.

6. Process for the preparation of agents for combating pests, characterised in that compounds of the formula (I), that is to say 3-(2,3-dichloro-3,3-difluoroprop-1-en-1-yl)-2,2-dimethylcyclopropanecarboxylic acid fluorobenzyl esters, are mixed with extenders and/or surface-active agents.